# EUROPÄISCHE PATENTANMELDUNG

(11) **EP 1 842 523 A1**
(43) Veröffentlichungstag der Anmeldung: **10.10.2007**
(21) Anmeldenummer: 07003719.7
(22) Anmeldetag: 23.02.2007
(51) Int. Cl.: A61K 8/11, A61K 8/19, A61K 8/23, A61K 8/26, A61Q 1/02, F41H 3/00

(54) **Tarnschminke**

(30) Priorität: 07.04.2006 DE 102006016423
(71) Anmelder: EADS Deutschland GmbH, 85521 Ottobrunn (DE)
(72) Erfinder: Obkircher, Bernt, 88263 Horgenzell (DE); Meisel, Thomas, 88697 Bermatingen (DE)
(74) Vertreter: Meel, Thomas

(57) **Zusammenfassung**

Die Erfindung betrifft eine Schminke zur Tarnung unbedeckter Körperteile im optischen und Infraroten Wellenlängenbereich, bestehend aus einer Grundsubstanz aus Fett und/oder Wachs und/oder Paraffin, wobei in die Grundsubstanz Tarnteilchen als Reflektoren für Infrarotstrahlung eingelagert sind. Erfindungsgemäß wirken die Tarnteilchen im Optischen dunkel, wobei die Tarnteilchen einen Kern aus Cu oder Al oder Ag oder Au oder Ni oder Messing oder Bronze mit nichtglatter Oberfläche aufweisen, der von einer Oberflächenschicht aus einem Sulfid bedeckt ist.

## Beschreibung

Die Erfindung betrifft eine Schminke zur Tarnung unbedeckter Körperteile im optischen und infraroten Wellenbereich. Sie umfasst als wesentliche Bestandteile eine Schminkengrundsubstanz aus Fett und/oder Wachs und/oder Paraffin und/oder Vaseline sowie darin eingelagerte Tarnteilchen.

Personen, wie z. B. Soldaten, die vor einem bestimmten Hintergrund nur schwer entdeckt werden wollen, benutzen entsprechend gefärbte/gemusterte Kleidung und für die unbedeckten Hautstellen - wie vor allem das Gesicht - entsprechend gefärbte Tarnschminke. Braune, grüne und schwarze Farbtöne sind üblich. Bei Verwendung von Wärmebildgeräten im 3-5 µm oder 8-12 µm Bereich ist diese Art Tarnschminke aber nicht wirksam, d. h. die geschminkten Hautstellen sind, weil sie Wärmestrahlung entsprechend der Körpertemperatur abstrahlen, im Wärmebildbereich leicht zu detektieren. Da die Kleidung die Oberflächentemperatur der Personen reduziert, bleibt daher als tarnerischer Schwachpunkt beim Soldaten das Gesicht übrig.

Bekannt sind niedrigemittierende Farben oder low emissive paints (WO 95/10 569), deren Tarneffekt im Infraroten darauf beruht, dass die Farbe neben Binder und einer thixotropen Substanz auch Pigmente und strahlungsreflektierende Teilchen enthält, wobei den Pigmenten eine besonders kleine Korngröße verliehen wird. Diese Pigmentverkleinerung ist sehr aufwendig. Darüber hinaus sind solche Farben für Schminkzwecke nicht zu gebrauchen, weil sie auf der Haut unverträglich und nur schwer wieder zu entfernen sind (Binder, thixotrope Substanz, Lösungsmittel). Auch die besonders geringe Korngröße der Pigmente (< 0,1 µm) widerspricht der Anwendung auf der Haut, weil sich die Pigmente in die Poren setzen würden und praktisch nicht entfernt werden könnten.

In der US 5,254,406 A1 ist eine Tarnschminke offenbart, bei der als Grundsubstanz ein Wachs eingesetzt wird, in das Metallpartikel eingelagert sind. Diese Schminke wirkt wegen dieser Metallpartikel optisch jedoch hell und ist im optischen Bereich daher zur Tarnung ungeeignet.

Von der DE 198 27 989 A1 ist eine weitere Tarnschminke bekannt, die sowohl im Optischen als auch im Infraroten wirksam ist. In die Grundsubstanz aus Fett, Wachs etc. sind Metallteilchen als IR-Reflektoren eingelagert. Die Wirkung im Optischen wird durch Pigmente erzeugt, die oberhalb 0,7 µm eine Bandkante aufweisen, so dass sie im Optischen dunkel wirken. Die Pigmente sind jedoch nicht vollständig IRtransparent. Sie erhöhen somit das Emissionsvermögen im Infraroten, was die Tarnwirkung in diesem Wellenlängenbereich vermindert. Außerdem hat sich ergeben, dass die Hautverträglichkeit nicht optimal ist.

In der DE 12 87 011 B sind anorganische Pigmente für Tarnfarben beschrieben, die mit organischen Farbstoffen überzogen sind. Sie sind zur Tarnung sowohl im optischen wie auch im Infraroten Wellenlängenbereich vorgesehen.

Aufgabe der Erfindung ist, eine Tarnschminke zu schaffen, die sowohl im Bereich des sichtbaren Lichts als auch im Infraroten eine verbesserte Tarnwirkung ermöglicht.

Diese Aufgabe wird mit dem Gegenstand des Patentanspruch 1 gelöst. Vorteilhafte Ausführungen der Erfindung sind Gegenstand von Unteransprüchen.

Der erfindungsgemäßen Schminke aus Grundsubstanzen auf Fett-, Wachs- und/oder Paraffinbasis sind metallische Tarnteilchen beigemischt, die sowohl als diffuse, reflektorische Zentren für Umgebungsstrahlung im Infraroten wirken als auch aufgrund ihrer dunklen Farbe im sichtbaren (optischen) Bereich als Tarnung wirksam sind. Vorteilhafterweise beträgt der Anteil der Tarnteilchen an der Tarnschminke 40 - 95 Gew.%.

Die kombinierte Wirkung der Tarnteilchen im infraroten und im optischen Bereich wird dadurch ermöglicht, dass die Tarnteilchen einen metallischen Kern aufweisen mit einer sehr dünnen sulfidischen Oberflächenschicht, die den Teilchen die gewünschte dunkle Farbe im Optischen verleiht. Die Dicke der Oberflächenschicht wird dabei insbesondere geringer als 1µm und in einer optimalen Ausführung im Bereich kleiner als 1µm und größer als 500nm gewählt. Da die Wellenlänge von IR-Strahlung etwa 10 bis 20 mal größer ist als die Wellenlänge des optischen Lichts, ist die Oberflächenschicht im Infraroten praktisch durchsichtig, so dass im Infraroten das darunter liegende Metall als reflektorisches Zentrum für Umgebungs-IR-Strahlung wirkt.

Insgesamt wirkt somit die dunkle Oberflächenschicht der Tarnteilchen im Optischen und der metallische Kern der Tarnteilchen im Infraroten. Somit wird in beiden Wellenbereichen eine gute Tarnungswirkung erzielt.

Die Oberfläche der Tarnteilchen bzw. des Kerns sollte eine nichtglatte Oberfläche aufweisen. Eine glatte Oberfläche würde nämlich zu verstärkten Glanzeffekten im optischen Bereich führen, die sich naturgemäß störend auf den Tarneffekt auswirken. Insbesondere die in der dekorativen Kosmetik weit verbreiteten plättchenförmigen Glanzpigmente (siehe z.B. DE 42 23 383 A1) sind für einen erfindungsgemäßen Einsatz grundsätzlich nicht geeignet. Die erfindungsgemäß verwendeten Kerne (im Ausgangszustand in Pulverform vorliegend) weisen typischerweise eine äußere Form auf, bei deren räumlicher Ausdehnung keine der drei Dimensionen signifikant bevorzugt ist. Die Tarnteilchen weisen bevorzugt einen Durchmesser von mehr als 10 µm auf.

Als Kernmaterialien für die Tarnteilchen werden erfindungsgemäß Kupfer, Aluminium, Silber, Gold, Nickel, Messing oder Bronze verwendet. Es ist auch möglich, in der Tarnschminke Tarnteilchen unterschiedlicher Kernmaterialien einzusetzen, wobei als Kernmaterialien die bereits genannten Stoffe zur Anwendung kommen.

Erfindungsgemäß werden für die Oberflächenschicht sulfidische Materialien verwendet. Sie werden bevorzugt durch eine chemische Reaktion an der Oberfläche des Kernmaterials mit einem Reaktionspartner hergestellt.

Hergestellt werden solche Tarnteilchen zum Beispiel aus einem Kupferpulver, das in etwa 80°C warme Schwefellauge (schwach alkalische Lösung von sog. Schwefelleber, einer Mischung aus Kaliumsulfid, Kaliumsulfat und Kaliumthiosulfat, die üblicherweise zum Beizen von Kupferoberflächen benutzt wird) geschüttet wird. Dabei beginnen sich die Kupferteilchen an der Oberfläche dunkel zu färben, in dem Maße, wie Kupfersulfid an der Oberfläche entsteht. Der Sulfidierungsprozess kann gestoppt werden, wenn der gewünschte Grad der Dunkelfärbung erreicht ist. Dazu wird die Schweflelleberkonzentration und /oder die Prozesstemperatur durch Zumischen von Wasser erniedrigt. Besonders geeignet sind Kupferpulver mit einem Durchmesser größer als 10 µm und einer darauf erzeugten Kupfersulfidschicht kleiner als 1 µm.

Vorteilhaft sind bei der Auswahl der für die Grundsubstanz der Tarnschminke einzusetzenden Fette, Wachse, Paraffine (z.B. Vaselin) inklusive deren Hilfsstoffe, wie z.B. Konservierungsmittel, Konsistenzgeber usw, diejenigen zu bevorzugen, die im Infraroten, vor allem bei 8-12 µm, eine möglichst hohe Transparenz aufweisen, insbesondere mit einem Emissionskoeffizienten ε <0,5.

Die erfindungsgemäße Tarnschminke ermöglicht eine verbesserte Tarnung sowohl im Optischen als auch gegen Aufklärung durch Restlichtverstärker, Wärmebildgeräte (welche insbesondere im Wellenlängenbereich von 3-5 µm oder 8-12 µm arbeiten) und andere, im Infraroten wirksame Systeme. Sie ist außerdem hautverträglich.

## Patentansprüche

1. Schminke zur Tarnung unbedeckter Körperteile im optischen und Infraroten Wellenlängenbereich, bestehend aus einer Grundsubstanz aus Fett und/oder Wachs und/oder Paraffin, wobei in die Grundsubstanz Tarnteilchen als Reflektoren für Infrarotstrahlung eingelagert sind, **dadurch gekennzeichnet, dass** die Tarnteilchen im Optischen dunkel wirken, wobei die Tarnteilchen einen Kern aus Cu oder Al oder Ag oder Au oder Ni oder Messing oder Bronze mit nichtglatter Oberfläche aufweisen, der von einer Oberflächenschicht aus einem Sulfid bedeckt ist.

2. Schminke nach Anspruch 1, **dadurch gekennzeichnet, dass** sie 40 bis 95 Gew.% Tarnteilchen enthält.

3. Schminke nach Anspruch 1 oder 2, **dadurch gekennzeichnet, dass** die Grundsubstanz eine Mischung aus Tarnteilchen unterschiedlicher Kernmaterialien enthält.

4. Schminke nach einem der vorangehenden Ansprüche, **dadurch gekennzeichnet, dass** die sulfidische Oberflächenschicht der Tarnteilchen dünner als 1µm ist.

5. Schminke nach Anspruch 4, **dadurch gekennzeichnet, dass** die sulfidische Oberflächenschicht dicker als 500nm ist.

6. Schminke nach Anspruch 1, **dadurch gekennzeichnet, dass** die sulfidische Oberflächenschicht durch chemische Reaktion des Materials des Kerns mit einem Reaktionspartner hergestellt wird.

7. Schminke nach Anspruch 1, **dadurch gekennzeichnet, dass** die sulfidische Oberflächenschicht **dadurch** hergestellt wurde, dass ein Kupferpulver einer ca. 80°C warmen Schwefellauge zugegeben wird.

8. Schminke nach einem der vorangehenden Ansprüche, **dadurch gekennzeichnet, dass** die Tarnteilchen eine durchschnittliche Größe von mehr als 10 µm aufweisen.

9. Schminke nach einem der vorangehenden Ansprüche, **dadurch gekennzeichnet, dass** die Grundsubstanz eine hohe Transparenz im IR-Bereich von 8 bis 12 µm aufweist.
